(19) Europäisches Patentamt

European Patent Office

Office européen des brevets

(11) Publication number: **0 562 136 A1**

## EUROPEAN PATENT APPLICATION

(21) Application number: **92105058.9**

(22) Date of filing: **24.03.92**

(51) Int. Cl.⁵: **A61K 39/12, A61K 39/23**

(43) Date of publication of application:
**29.09.93 Bulletin 93/39**

(84) Designated Contracting States:
**DE FR**

(71) Applicant: **DIVISION OF MICROBIOLOGY, KYOTO BIKEN LABORATORIES, INC.**
**24-16, Makishima-cho**
**Uji-shi, Kyoto 611(JP)**

(72) Inventor: **Izumida, Akihiro**
**68-4 Tachikawa, Ujidawara-cho**
**Tsuzuki-gun, Kyoto 610-02(JP)**
Inventor: **Kodama, Kazuo**
**4-69 Oriidai 1-chome**
**Uji-shi, Kyoto 611(JP)**
Inventor: **Kubota, Michio**
**1-90 Ujinogami**
**Uji-shi, Kyoto 611(JP)**
Inventor: **Matsui, Osamu**
**48-20 Ujiichiban**
**Uji-shi, Kyoto 611(JP)**
Inventor: **Ishida, Yasuhisa**
**95-2 Murasakinodaitokuji-cho**
**Kita-ku, Kyoto-shi, Kyoto 603(JP)**

(74) Representative: **VOSSIUS & PARTNER**
**Postfach 86 07 67**
**D-81634 München (DE)**

(54) **Live vaccine to getah virus infectious disease, and trivalent live vaccine to Japanese encephalitis virus, porcine parvovirus and getah virus infectious diseases.**

(57) A trivalent live vaccine to Japanese encephalitis virus, porcine parvovirus and Getah virus infectious diseases, which comprises a mixture of (i) a live vaccine to Getah virus infectious disease as created by cultivating an attenuated Getah virus KB/VT strain, as obtained by continuous serial passages of a virulent Getah virus strain in Vero cells to 70 generations at 30°C followed by two times cloning by a plaque method to get purified virus, to HAL cells in such a way that the multiplicity of infection (amount of inoculated viruses/number of cells) is about 0.1; adsorbing the viruses to the cells for 60 minutes at 37 °C; then removing the inoculated viral fluid from the cells; adding a culture medium for cultivation of the viruses thereto; incubating the cells for 48 to 72 hours at 30°C; and, after the cytopathic effect (CPE) has been confirmed to progress to the middle degree or more, collecting the culture medium fluid to obtain an intended living vaccine to Getah virus infectious disease, (ii) a viral fluid as obtained by inoculating an attenuated Japanese encephalitis virus m strain to HmLu-1 cells as admitted to be suitable for propagation of the attenuated Japanese encephalitis virus m strain therewith, and (iii) a viral fluid as obtained by inoculating an attenuated porcine parvovirus HT⁻/SK strain to swine kidney culture cells as admitted to be suitable for propagation of the attenuated porcine parvovirus HT⁻/SK strain therewith, in such a proportion that the viral content in each viral fluid in the mixture is almost the same.

Rank Xerox (UK) Business Services
(3.10/3.6/3.3.1)

The present invention relates to a live vaccine to Getah virus infectious disease and to a trivalent live vaccine to Japanese encephalitis virus, Porcine parvovirus and Getah virus infectious diseases.

Recently, it has been found that infection of pregnant sows with Getah virus causes death of fetuses from them. Swines are animals having high sensitivity to Japanese encephalitis virus. In particular, when a pregnant sow is infected with the virus, fetuses in it are also infected therewith through its placenta to cause fetal death. In addition, infection of a pregnant sow with porcine parvovirus also results in infection of its fetuses therewith, like the case of Japanese encephalitis virus, to cause a problem of stillbirth.

In order to solve the problems, vaccines have already been developed to Japanese encephalitis virus and porcine parvovirus infectious diseases and have already been put to practical use. However, to Getah virus infectious disease, a vaccine is not developed up to the present, and development of it is earnestly desired. The number of sows or gilts for which are now bred here in Japan is presumed to be about 1,200,000, and vaccination of these swines against Japanese encephalitis virus, porcine parvovirus and Getah virus infectious diseases must be completed every year within a determined period before an epizootic of the diseases. For this, much labor which is almost beyond imagining is necessary for maintaining the swines and for preparing and sterilizing injectors and other injection appliances to be used and also for actually effecting injection to them for vaccination. Anyhow, the vaccination therefore needs large economical expenses.

In view of the above-mentioned points, the object of the present invention is to provide an attenuated Getah viral live vaccine to Getah virus infectious disease, which may confer a permanent immunity on swings against the disease by single injection thereof to swines, and to also provide trivalent live vaccine to Japanese encephalitis virus, porcine parvovirus and Getah virus infectious diseases. Using the vaccines, labor for maintaining swines to be vaccinated therewith may be reduced and also labor for preparing injectors or other injection appliances to be used for vaccination with them as well as labor for effecting injection of swines with them may be reduced, and therefore the economical expenses for vaccination of swines against the infectious diseases may be much reduced.

The live vaccine to Getah virus infectious disease of the present invention comprises an establishment of attenuated viral strain as obtained by serial passages of wild type Getah virus strain in Vero cells at a low temperature under permissive temperature range for propagation of it to obtain attenuated Getah virus KB/VT strain followed by incubating the KB/VT strain in HAL cells. In the case, a Getah virus virulent strain is passaged continuously in Vero cells at 30 °C for attenuating it. In addition, the serial passage is conducted continuously to 70 generations, followed by two times clonings by a plaque method to obtain attenuated KB/VT strain. The thus attenuated Getah virus KB/VT strain by such serial passages at permissive temperature for propagation of it is cultivated in HAL cells in such a way that the multiplicity of infection (M.O.I.; amount of inoculated viruses/number of cells) is about 0.1. After adsorption for 60 minutes at 37 °C, the inoculated viral fluid is removed, a culture medium for cultivation of viruses is added, and the cells are incubated for 48 to 72 hours at 30 °C. After the cytopathic effect (CPE) has been confirmed to progress to the middle degree or more, the culture fluids is collected to obtain a living vaccine to Getah virus infectious disease.

The trivalent live vaccine to Japanese encephalitis virus, porcine parvovirus and Getah virus infectious diseases of the present invention comprises a mixture of a viral fluid as obtained by inoculating an attenuated Japanese encephalitis virus m strain to HmLu-1 cells as admitted to be suitable for propagation of the attenuated Japanese encephalitis virus m strain therewith, a viral fluid as obtained by inoculating an attenuated porcine parvovirus $HT^-$/SK strain to swine kidney culture cells as admitted to be suitable for propagation of the attenuated porcine parvovirus $HT^-$/SK strain therewith, and a viral fluid as obtained by inoculating an attenuated Getah virus KB/VT strain to HAL cells as admitted to be suitable for propagation of the attenuated Getah virus KB/VT strain therewith, each in a suitable amount.

The attenuated Getah virus KB/VT strain is obtained from 2078 strain (an wild type strain of Getah virus as isolated from Culex tritaeniorhynchus and passaged to seven generations in a suckling mouse brain) by the serial passages in Vero cells to 70 generations, then cloning it two times by a plaque method, and further passaged it to one generation in HAL cells. The attenuated strain thus obtained by the process is used as an master seed virus, and the seed virus for vaccine production is prepared from master seed by the serial passage within limited passage generations. In general, in creating an attenuated strain, a means of serial passages of the virus at a low permissive temperature for propagation of it to attain the intended attenuation is employed. In the case of the Getah virus in the present invention, the virus is attenuated by continuous serial passages of it in Vero cells at 30 °C. The attenuated strain (KB/VT strain) thus obtained is not pathogenic even when it is inoculated in the brain of a suckling mouse. In addition, it forms clear small-sized plaques in cultivation with HAL cells and is easily differentiated from the parent strain of forming large-sized plaques. Such characteristics of the attenuated strain have been found to be stable even after serial passages thereof to five generations at 37 °C.

The above-mentioned attenuated Japanese encephalitis virus m strain is obtained by cultivating a strain derived from a virulent Japanese encephalitis virus Mukai strain, which is propagated in hamster kidney culture cells, by cloning, in mouse fetal fibroblasts, followed by the serial passages of the hamster kidney culture cells to ten generations and then cloning them. The seed virus thus obtained by the process is used as a master seed virus, which is further passaged to prepare a seed virus.

The above-mentioned attenuated porcine parvovirus HT⁻/SK strain is established by the serial passages of an attenuated strain (HT ⁻ strain), which is established by serial passage of 90HS strain (virulent strain of porcine parvovirus, as isolated from a stillborn swine fetus and passaged to eleven generations with ESK cells (established swine kidney cell line)) to 55 generations, to further 41 generations with swine kidney culture cells at 30 to 32°C. The thus obtained strain is used as a master seed virus, which is further passaged to prepare a seed virus.

The number of generations is to be three generations or less for the preparation of the master seed virus and two generations or less for the seed virus, in all the attenuated Japanese encephalitis virus m strain, attenuated porcine parvovirus HT⁻/SK strain and attenuated Getah virus KB/VT strain.

The proportion of the components of the attenuated Japanese encephalitis viral fluid, the attenuated porcine parvoviral fluid and the attenuated Getah viral fluid in the combined live vaccine is determined on the basis of the viral infective titer in each viral fluid. In general, the components may be blended to form the combined live vaccine in such a way that the viral contents in the respective viral fluids are almost same or the ratio of them is about 1/1/1/.

The live vaccine to Getah virus infectious disease of the present invention, as mentioned above, may be inoculated to sows and others to thereby produce an immune antibody for capable of obtaining a permanent immunity by single injection of the vaccine. In the case, the farrowing results are all those of normal birth, and all the piglets before drinking the colostrum from the dam are not infected with Getah virus. Therefore, inoculation of the vaccine of the present invention to pregnant sows causes no problems and is safe. Regarding the trivalent vaccine as prepared by combining the live vaccine to Getah virus infectious disease and Japanese encephalitis virus and porcine parvovirus, swines may be immunized to infections with Japanese encephalitis virus, porcine parvovirus and Getah virus by only single injection. Moreover, using the trivalent vaccine of the present invention, labor and economical expenses for vaccination of a large amount of swines within a determined period of time may be reduced noticeably.

Detailed Description of the Preferred Embodiments:

(1) Preparation of live vaccine of attenuated Japanese encephalitis virus:

An attenuated Japanese encephalitis virus m strain is inoculated to HmLu-1 cells so that M.O.I is about 0.1. After adsorbed for 60 minutes at 37°C, the inoculated viral fluid is removed, a culture medium fluid for propagation of the viruses is added, and the cultures are incubated for 24 to 48 hours at 37°C. After the cytopathic effect (CPE) has been confirmed to propagate to the middle degree or more, the culture fluids is collected.

(2) Preparation of live vaccine of attenuated porcine parvovirus:

For preparing attenuated porcine parvoviruses,HT-/SK strain is inoculated to swine kidney culture cells so that M.O.I. is about 0.1; and after adsorbed for 60 minutes at 37°C, the inoculated viral fluid is removed, a culture medium fluid for propagation of the viruses is added, the cultures are incubated for 7 days at 32°C, and the culture fluid is collected.

(3) Preparation of live vaccine of attenuated Getah virus:

For preparing attenuated Getah viruses, KB/VT strain is inoculated to HAL cells so that M.O.I. is about 0.1; and after adsorbed for 60 minutes at 37°C, the inoculated viral fluid is removed, a culture medium fluid for propagation of the viruses is added, and the cultures are incubated for 48 to 72 hours at 30°C. After the cytopathic effect (CPE) has been confirmed to propagate to the middle degree or more, the culture fluid is collected.

(4) Preparation of trivalent live vaccine to Japanese encephalitis virus, porcine parvovirus and Getah virus infectious diseases:

The viral fluids as obtained from each of the above-mentioned culture fluids has been examined and confirmed to be suitable as a material for preparation of vaccine, and all the viral fluids are mixed in a proportion of the attenuated Japanese encephalitis viral fluid to the attenuated porcine parvoviral fluids to the attenuated Getah viral fluid of being 1/1/1. To the mixture is added a stabilizer of an aqueous solution containing 20 w/v % lactose and 0.6 w/v % polyvinyl pyrrolidone in a proportion of being 1/2. The combined vaccine is divided in vials each in a suitable amount thereof, and freeze-dried.

(5) Safety and effectiveness:

In order to examine the safety of the trivalent vaccine thus prepared as above, the dried vaccine was dissolved in a phosphate-buffered saline solution to prepare a vaccine solution, which was inoculated to experimental animals.

(A) The vaccine was inoculated to 35 day-old mice subcutaneously, intramuscularly or intraperitoneally.

The results are shown in Table 1.

Table 1

(A) Mice

| Test Group | Mice Age (day-old) | Number of Mice tested | Inoculation Route | Amount Inoculated ml (strain) (*) TCID$_{50}$ | | Clinical Remarks | Mean Weight (g) before inoculation | Mean Weight (g) 10 days later |
|---|---|---|---|---|---|---|---|---|
| I | 35 | 10 | subcutaneous | 0.2ml | m $10^{5.1}$ | Normal | 20.5 | 26.9 |
| | | | | | H $10^{4.6}$ | | | |
| | | | | | K $10^{5.8}$ | | | |
| II | 35 | 10 | intramuscular | 0.2ml | m $10^{5.1}$ | Normal | 20.7 | 27.6 |
| | | | | | H $10^{4.6}$ | | | |
| | | | | | K $10^{5.8}$ | | | |
| III | 35 | 10 | intraperitoneally | 0.5ml | m $10^{5.5}$ | Normal | 21.1 | 27.2 |
| | | | | | H $10^{5.0}$ | | | |
| | | | | | K $10^{6.2}$ | | | |

(*) m : Attenuated Japanese encephalitis virus m strain

H : Attenuated porcine parvovirus HT-/SK strain

K : Attenuated Getah virus KB/VT strain

(B) The vaccine was inoculated to guinea pigs each having a weight of about 300g subcutaneously, intramuscularly or intraperitoneally. The results are shown in Table 2.

Table 2

(B) Guinea Pigs

| Test Group | Guinea Pigs Weight | Number of Test Animals | Inoculation Route | Amount Inoculated ml | strain | (*) TCID$_{50}$ | Clinical Remarks | Mean Weight (g) before inoculation | Mean Weight (g) 10 days later |
|---|---|---|---|---|---|---|---|---|---|
| I | 310 | 3 | subcutaneous | 2.0ml | m | $10^{6.1}$ | Normal | 310 | 385 |
| | | | | | H | $10^{5.6}$ | | | |
| | | | | | K | $10^{6.8}$ | | | |
| II | 313 | 3 | intramuscular | 1.0ml | m | $10^{5.8}$ | Normal | 313 | 373 |
| | | | | | H | $10^{5.3}$ | | | |
| | | | | | K | $10^{6.5}$ | | | |
| III | 307 | 3 | intraperitoneally | 5.0ml | m | $10^{6.5}$ | Normal | 307 | 380 |
| | | | | | H | $10^{6.0}$ | | | |
| | | | | | K | $10^{7.2}$ | | | |

(*) m : Attenuated Japanese encephalitis virus m strain

H : Attenuated porcine parvovirus HT⁻/SK strain

K : Attenuated Getah virus KB/VT strain

As is obvious from the results in Tables 1 and 2, all the tests animals were healthy and the safety of the above-mentioned combined vaccine was proved.

(C) The trivalent vaccine was inoculated to 7-day old piglets each having no immune antibody to Japanese encephalitis virus, porcine parvovirus and Getah virus. These piglets were observed and the results of them are shown in Table 3 below. All the tested piglets were healthy and the safety of the vaccine

5

was also proved.

Table 3

(C) Safety Test to piglets

| Tested piglets No. | Weight(kg) | Combined Vaccine Inoculation Route | Amount (mℓ) | Clinical Observation (days) 1 | 2 | 3 | 4 | 5 | 6 | 7 | 8 | 9 | 10 | 11 | 12 | 13 | 14 | Weight 14 days after inoculation(kg) |
|---|---|---|---|---|---|---|---|---|---|---|---|---|---|---|---|---|---|---|
| 1 | 2.4 | subcutaneous | 1.0 | - | - | - | - | - | - | - | - | - | - | - | - | - | - | 5.1 |
| 2 | 2.2 | | | - | - | - | - | - | - | - | - | - | - | - | - | - | - | 5.0 |
| 3 | 2.5 | | | - | - | - | - | - | - | - | - | - | - | - | - | - | - | 5.6 |
| 4 | 2.4 | | | - | - | - | - | - | - | - | - | - | - | - | - | - | - | 5.4 |
| 5 | 2.3 | control (not inoculated) | | - | - | - | - | - | - | - | - | - | - | - | - | - | - | 5.2 |

(D) In order to examine the safety of the vaccine to pregnant sows, the vaccine was injected to two pregnant sows which were then observed until they farrowed. The results of the observation are shown in Table 4, from which it is noted that all the tested sows were healthy. After inoculation of the vaccine, the sows were admitted to propduce an immune antibody to Japanese encephalitis virus, porcine parvovirus

6

and Getah virus. The farrowing results from the tested pregnant sows were all normal. Each piglet was bled before the taking of colostrum from dam and the blood from piglet was examined as to whether it had an immune antibody to Japanese encephalitis virus, porcine parvovirus and Getah virus. As a result, the blood was found to have no immune antibody to the viruses.

From this, it is proved that injection of the trivalent vaccine causes no fetal infection therewith in pregnant sows and is therefore safe to them.

Table 4

(D) Safety Test to Pregnant Pigs

| Tested sows No. | Pregnancy period (days) | Vaccine Inoculation Route | Dose (TCID$_{50}$)(*2) | Antibody titer (*1) before inoculation | Antibody titer (*1) after farrowing | Farrowing Results number of births | number of normal births | number of fetal deaths | Antibody of piglets before taking of colostrum (No. of positive/No. of tested) |
|---|---|---|---|---|---|---|---|---|---|
| 1 | 25 | subcutaneous | 100 ml $J10^{8.1}$ $H10^{7.5}$ $K10^{8.2}$ | J<10 P<10 G<10 | 1280 640 1280 | 11 | 11 | 0 | 0/11 |
| 2 | 28 | subcutaneous | 100 ml $J10^{8.1}$ $H10^{7.5}$ $K10^{8.2}$ | J<10 P<10 G<10 | 640 320 1280 | 10 | 10 | 0 | 0/10 |
| 3 | 45 | subcutaneous | 1 ml $J10^{6.1}$ $H10^{5.5}$ $K10^{6.2}$ | J<10 P<10 G<10 | 80 40 160 | 9 | 9 | 0 | 0/9 |
| 4 | 43 | subcutaneous | 1 ml $J10^{6.1}$ $H10^{5.5}$ $K10^{6.2}$ | J<10 P<10 G<10 | 40 20 80 | 12 | 12 | 0 | 0/12 |

(*1) J : Japanese encephalitis virus HI antibody titer
P : Porcine parvovirus HI antibody titer
G : Getah virus HI antibody titer
m : Infective titer of attenuated Japanese encephalitis m strain

(*2) H : Infective titer of attenuated porcine parvovirus HT⁻/SK strain
K : Infective titer of attenuated Getah virus KB/VT strain

Table 5

(E) Effectiveness Test

| Tested sows | | Vaccine Inoculation | | Challenge by virulent strain | | | Antibody titer | | | Farrowing | | |
|---|---|---|---|---|---|---|---|---|---|---|---|---|
| No. | pregnancy period (days) | Route | Dose (TCID$_{50}$) | Strain(*) | pregnancy period (days) when challenge inoculation | method of challenge | before inoculation | At a time of challenge | After farrowing | number of births | number of normal births | number of fetal deaths |
| 1 | 23 | subcutaneous 1ml | m10$^{5.8}$ H10$^{6.3}$ K10$^{6.5}$ | 44 | | virulent Japanese encephalitis virus (subcutaneous) | J<10 P<10 G<10 | 160 80 640 | 1280 20 160 | 11 | 11 | 0 |
| 2 | 25 | subcutaneous 1ml | m10$^{5.8}$ H10$^{6.3}$ K10$^{6.5}$ | 46 | | virulent porcine parvoviru (intranasal) | J<10 P<10 G<10 | 80 40 160 | 40 320 40 | 10 | 10 | 0 |
| 3 | 5 | subcutaneous 1ml | m10$^{5.8}$ H10$^{6.3}$ K10$^{6.5}$ | 26 | | virulent Getah virus (subcutaneous) | J<10 P<10 G<10 | 80 40 160 | 20 40 320 | 9 | 9 | 0 |

(*) Amount of challenge viruses : Virulent Japanese encephalitis virus Furumoto strain 10$^{4.0}$ LD$_{50}$
Virulent porcine parvovirus 90HS strain 10$^{5.0}$ TCID$_{50}$
Virulent porcine Getah virus 2078 strain 10$^{5.4}$ LD$_{50}$

(E) In order to examine the effectiveness of the trivalent vaccine of the present invention, the vaccine was injected to three pregnant sows. Three weeks after the injection, virulent Japanese encephalitis virus (subcutaneously), virulent porcine parvoviruses (nasally) or virulent Getah viruses (subcutaneously) were inoculated to each of them. The injected sows were then observed and the results are shown in Table 5. As is noted therefrom, the farrowing results of them were all normal. From the results, it was proved that the trivalent vaccine was effective to prevention of fetal death to be caused by Japanese encephalitis virus, porcine parvovirus and Getah virus.

(F) In order to determine the effective virus titer in the trivalent vaccine of the present invention, the effectiveness, if any, of various combinations of various contents of attenuated Japanese encephalitis virus m strain, attenuated porcine parvovirus HT⁻/SK strain and attenuated Getah virus KB/VT strain in the trivalent vaccine was examined. The results are shown in Table 6 , from which no difference is admitted in the effectiveness within the range of combinations of $10^{6.0}$ to $10^{6.5}$ $TCID_{50}$/dose of attenuated Japanese encephalitis virus m strain, $10^{5.0}$ to $10^{5.5}$ $TCID_{50}$/dose of attenuated porcine parvovirus HT⁻/SK strain and $10^{5.5}$ to $10^{6.6}$ $TCID_{50}$/dose of attenuated Getah virus KB/VT strain.

(F) Inoculation Test to the piglets with Various Virus Contents in Vaccine

Table 6

| Virus Content in Vaccine (TCID$_{50}$) | | | No. of Tested piglets | HI Antibody Value | | | | | |
|---|---|---|---|---|---|---|---|---|---|
| m | H | K | | JEV before inoculation | JEV After 4 weeks | PPV before inoculation | PPV After 4 weeks | GV before inoculation | GV After 4 weeks |
| 5.8 | 5.3 | 6.5 | 1 | <10 | 40 | <10 | 40 | <10 | 160 |
| | | | 2 | <10 | 80 | <10 | 80 | <10 | 320 |
| 6.1 | 5.5 | 6.2 | 3 | <10 | 80 | <10 | 80 | <10 | 160 |
| | | | 4 | <10 | 160 | <10 | 40 | <10 | 80 |
| 6.0 | 5.0 | 6.6 | 5 | <10 | 40 | <10 | 80 | <10 | 160 |
| | | | 6 | <10 | 40 | <10 | 40 | <10 | 320 |
| 6.5 | 5.5 | 5.5 | 7 | <10 | 80 | <10 | 80 | <10 | 40 |
| | | | 8 | <10 | 160 | <10 | 160 | <10 | 80 |

(G) Next, ten-fold dilutions the trivalent vaccine were made and applied to determine the dose response at piglets. The results are shown in Table 7, from which antibody response was confirmed in the attenuated Japanese encephalitis virus m strain of being $10^{3.0}$ TCID$_{50}$/dose or more, the attenuated porcine parvovirus HT$^-$/SK strain of being $10^{2.0}$ TCID$_{50}$/dose or more, and the attenuated Getah virus KB/VT strain of being $10^{3.6}$ TCID$_{50}$/dose or more.

10

Table 7

(G) Inoculation Test to piglets with Various Virus Contents in Vaccine

| Combined Vaccine Dilutions | Virus Content in Vaccine Tested (TCID$_{50}$) | | | Number of Tested Piglets | HI Antibody Value | | | | | |
|---|---|---|---|---|---|---|---|---|---|---|
| | | | | | JEV | | PPV | | GV | |
| | | | | | before inoculation | After 4 weeks | before inoculation | After 4 weeks | before inoculation | After 4 weeks |
| | m | H | K | | | | | | | |
| $10^0$ | 6.0 | 5.0 | 6.6 | 1 | <10 | 160 | <10 | 160 | <10 | 320 |
| | | | | 2 | <10 | 80 | <10 | 40 | <10 | 160 |
| $10^{-1}$ | 5.0 | 4.0 | 5.6 | 3 | <10 | 40 | <10 | 20 | <10 | 40 |
| | | | | 4 | <10 | 20 | <10 | 40 | <10 | 80 |
| $10^{-2}$ | 4.0 | 3.0 | 4.6 | 5 | <10 | 10 | <10 | 10 | <10 | 40 |
| | | | | 6 | <10 | 20 | <10 | 20 | <10 | 20 |
| $10^{-3}$ | 3.0 | 2.0 | 3.6 | 7 | <10 | 40 | <10 | 40 | <10 | 10 |
| | | | | 8 | <10 | 40 | <10 | 40 | <10 | 20 |
| $10^{-4}$ | 2.0 | 1.0 | 2.6 | 9 | <10 | <10 | <10 | <10 | <10 | <10 |
| | | | | 10 | <10 | <10 | <10 | <10 | <10 | <10 |
| $10^{-5}$ | 1.0 | 0 | 1.6 | 11 | <10 | <10 | <10 | <10 | <10 | <10 |
| | | | | 12 | <10 | <10 | <10 | <10 | <10 | <10 |

## Claims

1. A live vaccine to Getah virus infectious disease, comprising an attenuated viral fluid as obtained by serial passages of virulent Getah virus strain at permissive temperature for propagation of it, to obtain attenuated Getah virus KB/VT strain followed by incubating the KB/VT strain in HAL cells.

2. The live vaccine to Getah virus infectious disease as claimed in claim 1, in which the virulent Getah virus has been attenuated by continuous serial passages of it in Vero cells at 30 °C.

3. The live vaccine to Getah virus infectious disease as claimed in claim 1 or 2, in which the virulent Getah virus has been attenuated by continuous serial passages of it to 70 generations followed by two times cloning by a plaque method.

4. The live vaccine to Getah virus infectious disease as claimed in claim 1, obtainable by cultivating an attenuated Getah virus KB/VT strain, as obtained by serial passages of a virulent Getah virus strain at permissive temperature for propagation of it, to HAL cells in such a way that the multiplicity of infection (amount of inoculated viruses/number of cells) is about 0. 1; adsorbing the viruses to the cells for 60 minutes at 37 °C; then removing the inoculated viral fluid from the cells; adding a culture medium fluid for incubation of the viruses thereto; incubating the cells for 48 to 72 hours at 30 °C; and, after the cytopathic effect has been confirmed to progress to the middle degree or more, collecting the culture fluid to obtain an intended living vaccine to Getah virus infectious disease.

5. A trivalent live vaccine to Japanese encephalitis virus, porcine parvovirus and Getah virus infectious diseases, comprising a mixture of a viral fluid obtainable by incubating an attenuated Japanese encephalitis virus m strain in HmLu-1 cells, a viral fluid obtainable by incubating an attenuated porcine parvovirus $HT^-$ /SK strain in swine kidney culture cells, and a viral fluid obtainable by incubating an attenuated Getah virus KB/VT strain in HAL cells.

6. The trivalent live vaccine to Japanese encephalitis virus, porcine parvovirus and Getah virus infectious diseases as claimed in claim 5, comprising a mixture of a viral fluid as obtained by incubating an attenuated Japanese encephalitis virus m strain in HmLu-1 cells, a viral fluid as obtained by incubating an attenuated porcine parvovirus $HT^-$ /SK strain in swine kidney culture cells, and a viral fluid as obtained by incubating an attenuated Getah virus KB/VT strain in HAL cells, in a proportion of 1/1/1.

7. The trivalent live vaccine to Japanese encephalitis virus, porcine parvovirus and Getah virus infectious diseases as claimed in claim 5 or 6, comprising combination of an attenuated Japanese encephalitis virus m strain of being $10^{3.0}$ $TCID_{50}$/dose or more, an attenuated porcine parvovirus $HT^-$ /SK strain of being $10^{2.0}$ $TCID_{50}$ /dose or more and an attenuated Getah virus KB/VT strain of being $10^{3.6}$ $TCID_{50}$/dose or more.

8. The trivalent live vaccine to Japanese encephalitis virus, porcine parvovirus and Getah virus infectious diseases as claimed in claim 5 or 6, comprising combination of an attenuated Japanese encephalitis virus m strain of being from $10^{6.0}$ to $10^{6.5}$ $TCID_{50}$/dose, an attenuated porcine parvovirus $HT^-$ /SK strain of being from $10^{5.0}$ to $10^{5.5}$ $TCID_{50}$/dose and an attenuated Getah virus KB/VT strain of being from $10^{5.5}$ to $10^{6.6}$ $TCID_{50}$ /dose.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 115, 1991, Columbus, Ohio, US; abstract no. 154936q, IZUMIDA, AKIHIRO ET AL. 'ESTABLISHMENT AND BIOLOGICAL PROPERTIES OF AN ATTENUATED STRAIN OF GETAH VIRUS' page 526 ; * abstract * & NIPPON JUISHIKAI ZASSHI vol. 44, no. 3, 1991, JAPAN pages 197 - 201 | 1-4 | A61K39/12 A61K39/23 |
| X | AGRIS    ABSTRACT 92-016307, IZUMIDA, A. ET AL.:"SAFETY AND IMMUNOGENICITY OF THE ATTENUATED KB/VT STRAIN OF GETAH VIRUS IN SWINE." & JOURNAL OF THE JAPAN VETERINARY MEDICAL ASOCIATION, APR 1991, V. 44(4) p.313-318. | 1-4 | |

TECHNICAL FIELDS SEARCHED (Int. Cl.5)

C12N
A61K

The present search report has been drawn up for all claims

| Place of search | Date of completion of the search | Examiner |
|---|---|---|
| THE HAGUE | 17 NOVEMBER 1992 | REMPP G.L.E. |